# EUROPEAN PATENT APPLICATION

(11) **EP 0 682 938 A1**
(43) Date of publication of application: **22.11.1995**
(21) Application number: 94830231.0
(22) Date of filing: 19.05.1994
(51) Int. Cl.: A61K 7/48

(54) **A cosmetic mask for beauty-care skin treatments, and process for making same**

(71) Applicant: OSMOS S.P.A., I-20129 Milano (IT)
(72) Inventor: Gentile, Giansergio, I-22060 Carimate (Como) (IT)
(74) Representative: Giambrocono, Alfonso, Dr. Ing.

(57) **Abstract**

A new cosmetic mask is described, in the matrix of which a natural rubber is included in a percentage ranging between 2% and 6%, to give the mask a malleable and elastic consistency. As compared to the products presently available on the market, the mask can be applied and removed more easily, does not soil, is not greasy and at the end of the treatment it is not necessary to clean the face, in that no traces of the product are left thereon.

## Description

The present invention relates to a cosmetic mask for beauty-care skin treatments, comprising: a water-base matrix containing preservating, moistening and stabilizing substances and a buffer agent; at least one active ingredient dissolved in said matrix arid selectively having a relaxing, decongesting, purifying, moistening, toning, astringent, brightening, moisturizing, red-relieving and anti-wrinkle function.

It is also an object of the present invention a process for making the above mask.

Cosmetic masks for body care and beauty treatments have been known since long but advices on their actual utility and their method of acting have been so far different. Thus on the one hand cosmetic masks are deemed to exclusively be a side method for skin cleansing, whereas on the other hand a great number of cosmetologists is convinced that a mask above all serves to cause a blood recall to the under-epidermal tract.

Independently of how they are judged, masks undoubtedly have specific well defined functions depending on the active ingredient (or active ingredients) contained therein. For example, they have an astringent, moisturizing, relaxing, nourishing, stimulating, toning, cleansing, brightening, red-relieving function, ecc.

In principle, masks can be divided into three general classes, that is clayey masks, masks in the form of a film and in the form of a cream.

Clayey masks appear in the form of a powder to be wetted at the moment of use or already in the form of a homogeneous mass. Once applied to the face for example, they dry in a few minutes and are eliminated with a wet sponge or abundant rinsing water.

Masks in the form of a film were in the past made of natural gums and mucilages, whereas they are presently made of synthesis vinyl polymers. After they are applied, these masks dry giving rise to a thin and transparent film that can be removed using fingertips.

Through the use of another type of resin not in the form of a film (a carboxyvinyl resin) or the use of cellulose ethers or natural polymers (alginates, carrageenates), undetachable gel-like masks are obtained that are usually employed to route brightening and refreshing substances. They are usually removed by a wet sponge or abundant rinsing water.

Finally, masks in the form of cream are normal emulsions into which the most different substances may be incorporated even at high concentrations. These masks do not dry and can be, in case of need, slightly massaged. As they mostly are oil-in-water emulsions, that is hydrodispersible emulsions, they are removed easily with the aid of tissue paper and cotton wool.

Depending on their function, masks in the form of cream can contain different active ingredients: thus, the moisturizing masks for example contain natural moisturizing factors, commercially referred to as "NMF", egg, carotenes; the red-relieving ones contain azulene, camomile, calendula: toning ones contain pollen, collagen, hop, ginseng; purifying ones contain sulphur, zinc oxide; astringent ones contain Krameria triandria, tormentil, aluminium salts; brightening ones contain hamamelis, cucumber, lemon.

Masks in the form of film generally are hamamelis-, chestnut- or ruscus-based masks, whereas clayey masks contain sulphur, zinc oxide, plant extracts, essential oils, etc.

All the above mentioned known masks have more or less important drawbacks and disadvantages. Thus, for example, in the case of a dry skin having oily portions, there is the possibility that during treatment part of the product applied to the dry portions reaches the oily portion and vice-versa, so that the desired effects will not be achieved. Clayey masks and masks in the form of cream are rather prone to soil or smear with grease either the user or the person assigned to the treatment, whereas those in the form of film need time to get dry in order to give origin to a film and these too can reach undesired points in the face or body.

In addition masks of known type contain emulsifying and surface-active agents that, being rather irritant, make it impossible to apply the masks themselves to particularly sensitive and delicate points, such as eyelids for example.

The present invention aims to provide a cosmetic mask that, after application, is capable of being removed without resorting to toning lotions, sponges, water or similar expedients, that does not form a film needing to dry before being removed and that does not spread out of the application point.

The invention also aims to provide a mask capable of being applied in a simple and easy fashion, even to very sensible and delicate points, which is malleable and of a lower cost than the products of same kind presently available on the market.

It has been found that a mask having a gummy, soft, malleable and elastic consistency, very similar to that of a chewing-gum once it has been moistened by saliva, can be applied in a very simple manner, does not soil, can be shaped at will on the part to which it is being applied and can be removed easily, once its function has been terminated. Such a mask, in addition, does not adhere fixedly to the skin and is agreeable to the touch and view.

It is therefore an object of the invention a cosmetic mask characterized in that said matrix further comprises 2 to 6% by weight of a natural rubber dispersed in said water base, which natural rubber gives the mask a gummy, soft, malleable and elastic consistency.

Undoubtedly the mask being the object of the invention represents an innovatory improvement in the field of the art, in that, in addition to offering advantages as compared to the masks already available on the market, does not fall within anyone of the above mentioned classes.

It is a further object of the present invention a process for making the cosmetic mask which is characterized in that it comprises five operating stages than can be also carried out simultaneously.

A first stage comprises the step, referred to as step A, of preparing a mixture consisting of water, a preservative and a binder.

A second stage comprises the step, referred to as step B, of preparing a mixture consisting of water and natural rubber.

A third stage comprises the step, referred to as step C, of preparing a mixture consisting of water, and stabilizing and chelating agents.

A fourth stage comprises the step, referred to as step D, of preparing a saturated mixture consisting of water and salts having stabilizing, thickening and plugging functions.

The last mentioned mixture may also be prepared in conjunction with the preparation of the mixture referred to at step C.

A fifth stage comprises the step, referred to as step E, of preparing a mixture consisting of water, a humectant, a preservative and an emollient agent.

Once the above listed five steps have been completed, which steps are usually carried out simultaneously in appropriate separate reactors, the mixture obtained in step A is poured into the one obtained in step B. Then mixtures obtained respectively in steps C, D and E are added to the composition thus obtained. At the end the active ingredients (or active ingredient) are added and pouring off into appropriate vessels is carried out, preferably at a temperature of approximately 30°C.

The gummy mass keeps malleable and elastic and does not exhibit separations or defects, even after long storage periods. The consistency of same does not undergo changes in time.

As preservatives, binders, stabilizers, humectants and additives in general, the usual esters of the 4-hydroxybenzoic acid and salts of alkaline and alkaline-earth metals can be taken into consideration, such as for example methyl and propyl hydroxybenzoates, silicates, phosphates and glucose and urea derivatives.

As the active ingredient, any substance used to this end in normal cosmetic masks can be employed, depending on requirements, provided that such a substance is water-soluble.

As above pointed out, the essential ingredient to give the final product the soft, malleable and elastic gummy consistency is a natural rubber.

Preferentially it is a gum selected from the group consisting of gum arabic, guar gum, Xanthorrhoea gum, tragacanth, cashew gum and karaja gum.

The amount of said gum in the mixture ranges between 2% and 6% by weight, referred to the overall weight of the matrix. The exact percentage of the gum used is established so as to give the desired viscosity to the final product.

As the term of evaluation for viscosity, the drainage time at room temperature (20-25°C) of a 200 g sample of the product from a funnel with an outlet diameter of 2 cm is assumed. This time is preferably 120 s and at all events included between 90 s and 180 s.

The active ingredients (or active ingredient) and optional colouring agents added, in the form of a glycolic or a glyceric solution respectively, to the matrix absolutely must not overcome a well determined amount, otherwise the mask will not become sufficiently viscous and will lose its essential features. A too small amount thereof is to be avoided as well, otherwise the mask function will not be able to be completely performed.

An amount ranging between 2% and 10% by weight referred to the overall mask weight has been found to be useful, the exact percentage being also established depending on the matrix viscosity.

A preferred cosmetic mask according to the present invention comprises: 70% to 80% by weight of deionized water, 0.5% to 2% by weight of Mg and Na silicate, 2% to 6% by weight of natural rubber, 0.5% to 5% by weight of monosodium phosphate, 3% to 6% by weight of dipotassium phosphate, 2% to 5.5% by weight of tricalcium phosphate, 2% to 7% by weight of glycerol, 1% to 6% by weight of methyl glucose 10 (OE), 0.2% by weight of methyl paraben (methyl hydroxybenzoate), 0.15% by weight of propyl paraben (propyl hydroxybenzoate), 0.2% by weight of EDTA tetrasodium salt (ethylenediaminetetraacetic acid tetrasodium salt), 0.3% by weight of imidazoledinylurea and 2% to 10% by weight of the active ingredient in a glycolic solution.

Obviously any person skilled in the art is capable of modifying the final composition of the mask, for example by varying the salts, preservatives or other additives or finding another natural rubber behaving as those mentioned in the description.

Any modification of this type completely falls within the scope of the present invention, which is hereinafter illustrated by means of an example which is given by way of indication only and not for the purpose of limiting the present invention.

### Example

For preparing a 100 kg matrix, 23.6 l of deionized water and 3.6 kg of guar gum are introduced into a turbine emulsifier also equipped with a mechanical stirring device, thermometer and electric heater and stirring is carried out for about 30 minutes at room temperature, as far as a homogeneous solution is achieved (step B).

Separately, in four reactors equipped with a stirrer, thermometer, heater and device for taking samples, the four other steps of the process are carried out by combination of the following ingredients:

| Step A: | |
|---|---|
| deionized water | 23.05 l |
| methyl paraben | 0.2 kg |
| propyl paraben | 0.15 kg |
| Mg and Na silicate | 1.0 kg |

The ingredients are constantly mixed for about 15 minutes, gradually heating the mixture until a temperature of about 80°C is reached.

| Step C: | |
|---|---|
| deionized water | 10.4 l |
| EDTA, tetrasodium salt | 0.2 kg |
| monosodium phosphate | 1.0 kg |

Said ingredients are constantly mixed for about 15 minutes, gradually heating the mixture until a temperature of about 50°C is reached.

| Step D: | |
|---|---|
| deionized water | 15.4 l |
| dipotassium phosphate | 5.2 kg |
| tricalcium phosphate | 4.4 kg |

| Step E: | |
|---|---|
| glycerol | 5.2 kg |
| methyl glucose 10 (OE) | 4.0 kg |
| deionized water | 2.3 l |
| imidazoledinylurea | 0.3 kg. |

The ingredients of steps D and E are constantly mixed for about 5 minutes at room temperature.

Then, under stirring and keeping a temperature of about 50°C and at all events preferably included between 30°C and 60°C, the mixture obtained in step A in poured into the mixture obtained in step B and, still under stirring, mixtures obtained in step C, step D and step E respectively are added in the order. For each of said adding operations an average stirring time of about 15 minutes is required. Subsequently, still under stirring for approximately further 15 minutes, the active ingredients are added; afterwards the product obtained is poured, preferably still hot, into appropriate storage vessels, from which it will be then taken for being packaged in the usual manner.

The finished product has a malleable and elastic consistency and is pleasant to the touch. It can be manipulated with the fingers without any problem and employed for the desired purposes. The mask can then be removed without difficulty and the face does not need to be either washed or cleaned, in that oil or residual-material traces are not noticed.

## Claims

1. A cosmetic mask for beauty-care skin treatments, comprising:
- a water-base matrix containing preservating, moistening and stabilizing substances and a chelating agent;
- at least one active ingredient dissolved in said matrix and selectively having a relaxing, decongesting, purifying, moistening, toning, astringent, brightening, moisturizing, red-relieving and anti-wrinkle function, characterized in that said matrix further comprises 2 to 6% by weight of a natural rubber dispersed in said water base, which natural rubber gives the mask a gummy, soft, malleable and elastic consistency.

2. A cosmetic mask according to claim 1, characterized in that it exhibits such a viscosity that for a 200 g sample of said mask, at a temperature included between 20°C and 25°C, the drainage time of the sample from a funnel with an outlet diameter of 2 cm ranges between 90 s and 180 s.

3. A cosmetic mask according to claim 1, characterized in that the natural rubber is selected from a group consisting of gum arabic, guar gum, Xanthorrhoea gum, tragacanth gum, cashew gum and karaja gum.

4. A cosmetic mask according to claim 3, characterized in that the natural rubber is guar gum.

5. A cosmetic mask according to claim 4, characterized in that the gum is present in an amount of 3.6% by weight, with reference to the overall matrix weight.

6. A cosmetic mask according to claim 1, characterized in that the amount of the active ingredient or active ingredients in the form of a glycolic solution, ranges between 2% and 10% by weight referred to the overall mask weight.

7. A cosmetic mask according to claim 1, characterized in that said matrix contains 70% to 80% of deionized water.

8. A cosmetic mask according to claim 7, characterized in that as the stabilizers the matrix contains:
- 0.5% to 5% by weight of monosodium phosphate,
- 3% to 6% by weight of dipotassium phosphate,
- 2% to 5.5% by weight of tricalcium phosphate.

9. A cosmetic mask according to claim 7, characterized in that as the binder the matrix contains 0.5% to 2% by weight of Mg and Na silicate.

10. A cosmetic mask according to claim 7, characterized in that as the preservatives the matrix contains methyl paraben, propyl paraben and imidazoledinylurea, the overall percentage by weight of which is included between 0.2% and 1%.

11. A cosmetic mask according to claim 7, characterized in that as the chelating agent the matrix contains EDTA tetrasodium salt the percentage by weight of which is included between 0.1% and 0.3%.

12. A cosmetic mask according to claim 7, characterized in that as the humectants the matrix contains:
- 2% to 7% of glycerol,
- 1% to 6% of methyl glucose.

13. A process for making a cosmetic mask for beauty-care skin treatments, characterized in that it comprises the following steps:
Step A) preparing a mixture comprising:
- deionized water
- methyl paraben
- propyl paraben
- Mg and Na silicate,
Step B) preparing a mixture comprising:
- deionized water
- natural rubber;
Step C) preparing a mixture comprising:
- deionized water
- EDTA tretrasodium salt
- monosodium phosphate;
Step D) preparing a mixture comprising:
- deionized water
- dipotassium phosphate
- tricalcium phosphate;
Step E) preparing a mixture comprising:
- glycerol
- deionized water
- methyl glucose 10 (OE)
- imidazoledinylurea;
Step F) pouring the mixture from step A into the mixture from step B;
Step G) adding to the mixture from step F, the mixtures obtained in steps C, D and E respectively, in the order;
Step H) adding to the matrix obtained from the preceding steps at least one active ingredient selectively having a relaxing, decongesting, purifying, moistening, toning, astringent, brightening, moisturizing, red-relieving and anti-wrinkle function,
the above steps being executed under stirring.

14. A process according to claim 13, characterized in that in step B the natural rubber is selected from the group consisting of gum arabic, guar gum, Xanthorrhoea gum, tragacanth gum, cashew gum and karaja gum.

15. A process according to claim 13, characterized in that in step B the natural rubber is added in an amount included between 2% and 6% by weight, referred to the overall weight of the matrix obtained from steps A to G.

16. A cosmetic mask according to claim 13, characterized in that the mixtures referred to in steps C and D are prepared jointly through blending of the respective ingredients.
